# EUROPEAN PATENT APPLICATION

(11) **EP 1 424 086 A1**
(43) Date of publication of application: **02.06.2004**
(21) Application number: 03254088.2
(22) Date of filing: 27.06.2003
(51) Int. Cl.: A61L 15/28

(54) **Hemostatic wound dressing containing aldehyde-modified polysaccharide and hemostatic agents**

(30) Priority: 26.11.2002 US 304472
(71) Applicant: ETHICON, INC., Somerville, NJ 08876 (US)
(72) Inventor: Pendharkar, Sanyog Manohar, Old Bridge, New Jersey 08857 (US); Gorman, Anne Jessica, Highstown, NJ 08520 (US)
(74) Representative: Mercer, Christopher Paul

(57) **Abstract**

The present invention is directed to hemostatic wound dressings that contain a substrate for contacting a wound, wherein the substrate includes a wound-contacting surface and is fabricated at least in part from a biocompatible aldehyde-modified polysaccharide having covalently conjugated there with a hemostatic agent, and to methods of providing hemostasis to a wound that include applying the wound dressing described herein to a wound.

## Description

### FIELD OF THE INVENTION

The present invention relates to hemostatic wound dressings containing or fabricated from an aldehyde-modified polysaccharide, e.g. aldehyde-modified regenerated cellulose, having covalently conjugated there with a hemostatic agent, and to a method of providing hemostasis to a wound.

### BACKGROUND OF THE INVENTION

The control of bleeding is essential and critical in surgical procedures to minimize blood loss, to reduce post-surgical complications, and to shorten the duration of the surgery in the operating room. Oxidized cellulose, due to its biodegradable, bactericidal, and hemostatic properties, has long been used as a topical hemostatic wound dressing in a variety of surgical procedures, including neurosurgery, abdominal surgery, cardiovascular surgery, thoracic surgery, head and neck surgery, pelvic surgery, and skin and subcutaneous tissue procedures.

The use of oxidized cellulose as a hemostat was first described by Virginia Franz in 1944. Currently available oxidized cellulose hemostats are knitted or non-woven fabrics comprising carboxylic oxidized cellulose. Oxidized regenerated cellulose (ORC) is carboxylic-oxidized cellulose comprising reactive carboxylic acid groups. Examples of ORC absorbable hemostats commercially available include Surgicel® absorbable hemostat, a knitted fabric of ORC; Surgicel Nu-Knit® absorbable hemostat, a dense ORC fabric; and Surgicel® Fibrillar absorbable hemostat; all available from Johnson & Johnson Wound Management Worldwide, a division of Ethicon, Inc., Somerville, New Jersey, a Johnson & Johnson Company. Other examples of commercial absorbable hemostats containing oxidized cellulose include Oxycel® absorbable cellulose surgical dressing from Becton Dickinson and company, Morris Plains, New Jersey.

Although the absorbency of body fluid and the hemostatic action of currently available oxidized cellulose hemostats are adequate for applications where mild to moderate bleeding is encountered, they are not known to be effective to prevent or stop severe bleeding of high volume and high blood flow rate where a relatively high volume of blood is lost at a relatively high rate, nor are they known to achieve rapid hemostasis. In such instances, e.g. arterial puncture, liver resection, blunt liver trauma, blunt spleen trauma, aortic aneurysm, bleeding from patients with over-anticoagulation, or patients with coagulopathies, such as hemophilia, etc., a higher degree of hemostasis is required quickly.

In an effort to achieve enhanced hemostatic properties, blood-clotting agents, such as thrombin, fibrin and fibrinogen have been combined with carriers or substrates. Aqueous solution of thrombin is routinely used with gelatin-based carriers to enhance hemostasis at a surgical wound site. Two component fibrin sealants, consisting of thrombin and fibrinogen/Factor XIII have been used as surgical hemostats in liquid form or as a solid patch in combination with collagen matrix.

Physiologically, coagulation represents the transformation of soluble fibrinogen into an insoluble fibrin network under the influence of thrombin, the key enzyme. During the normal clotting cascade, fibrinogen is cleaved by thrombin and forms fibrin that polymerizes to form a fibrin clot, which is further strengthened by cross-linking by Factor XIII. Use of fibrin sealants to a bleeding surface results in accelerated hemostasis and a sealing effect on the bleeding surface.

Thrombin is a coagulation factor associated with an extraordinary range of biological activities. Thrombin has direct effects on coagulation, such as activating platelets, forming fibrin, and activating various procofactors and pro-enzymes in the coagulation cascade. Its biological activity extends through anticoagulation, stimulation of fibrinolytic reactions, activation of peripheral blood cell populations, and regulation of vascular tone. In addition to initiating processes leading to the sealing of a wound, thrombin is also responsible, in its role as a growth factor, in stimulating repair to tissue damage associated with the wound itself.

Sakamoto et al. in JP60087225 describe immobilizing thrombin and Factor XIII on oxidized cellulose substrate through a dehydrating condensation reaction, again using the acid oxidation product of cellulose as a substrate. However, the acidic nature of carboxylic oxidized cellulose substrate could rapidly denature and inactivate acid sensitive proteins, including thrombin or fibrinogen, on contact. Much of the enzymatic activity of thrombin and Factor XIII could be lost during the reaction. This makes it difficult to use the carboxylic-oxidized cellulose as a carrier for thrombin, fibrinogen, fibrin, or other acid sensitive biologics and pharmaceutical agents.

Hemostatic wound dressings containing neutralized carboxylic-oxidized cellulose and protein based-hemostatic agents, such as thrombin, fibrinogen and fibrin are known. Neutralized carboxylic-oxidized cellulosic materials are prepared by treating the acidic carboxylic-oxidized cellulose with a water or alcohol solution of a basic salt of a weak organic acid to elevate the pH of the cellulosic material to between 5 and 8 by neutralizing the acid groups on the cellulose prior to addition of thrombin in order to make it thrombin compatible. A thrombin hemostatic patch was disclosed, wherein thrombin was added to an acidic carboxylic oxidized regenerated cellulose or other material in presence of an acid neutralizing agent, epsilon aminocaproic acid (EACA), to raise the pH of the material to a region where thrombin can perform as a hemostat. While such neutralized carboxylic-oxidized cellulose may be thrombin compatible, it is no longer bactericidal, because the anti-microbial activity of oxidized cellulose is due to its acidic nature.

Hemostatic agents such as thrombin, fibrinogen or fibrin, if not covalently combined with the substrate, may be rinsed away by blood at a wound site. Alternatively, the non-bonded free form of thrombin, fibrinogen or fibrin, may migrate into the blood stream and potentially cause severe thrombosis in procedures such as arterial puncture, liver resection, blunt liver trauma, blunt spleen trauma, aortic aneurysm, etc., where higher blood pressure and higher blood velocity is encountered. Therefore, caution must be taken to prevent thrombin from migrating to the blood stream.

The use of cotton gauze that has been modified by oxidation to contain aldehyde, and then further by carboxymethylation, sulfonation or phosphorylation, has been disclosed for use in wound dressings. However, such dressings are not hemostatic and contain functional groups such as carboxymethyl, sulfonyl or phosphonyl groups.

Methods of producing highly oxidized tri-carboxylic acid derivatives of cellulose as hemostatic materials, involving two-stage oxidation by successive processing with an iodine-containing compound and nitrogen oxides, has been disclosed in RU2146264 and IN159322. As disclosed in these disclosures, oxidized cellulosic materials were prepared by preliminary oxidation with metaperiodate or periodic acid to yield periodate-oxidized, dialdehyde cellulose to form the intermediate for forming OC. The dialdehyde cellulose intermediate then is further oxidized by NO₂ to yield the OC, which is suitable for use as a hemostatic, anti-microbial and wound healing agent. The disclosures do not, however, suggest or disclose that the periodate-oxidized, dialdehyde cellulose intermediate formed in the first stage oxidation may or should be used in the preparation of wound dressings, e.g. hemostatic wound dressings.

To date, however, aldehyde-modified cellulose has not been utilized in wound dressings to provide hemostasis. No method is taught in the prior art whereby a di-hydroxyl containing material such as cellulose is oxidized with periodate to form an aldehyde-modified regenerated cellulose substrate. Nor has it been taught to covalently conjugate an active hemostatic protein such as thrombin, fibrinogen or fibrin, with an aldehyde-modified regenerated cellulose substrate to create a hemostatic device.

It would be advantageous to provide an anti-microbial hemostatic wound dressing that not only exhibits improved hemostasis via the inclusion of hemostatic agents, such as thrombin, fibrinogen or fibrin, but does so without the risk of the hemostatic agents migrating into the blood stream where they could cause severe thrombosis.

### SUMMARY OF THE INVENTION

The present invention is directed to hemostatic wound dressings that contain a substrate for contacting a wound, wherein the substrate comprises a wound-contacting surface and is fabricated at least in part from a biocompatible aldehyde-modified polysaccharide; and the substrate further includes a hemostatic agent covalently conjugated with the aldehyde-modified polysaccharide. The invention also is directed to methods of providing hemostasis to a wound that includes applying the wound dressing described herein to a wound.

### DETAILED DESCRIPTION OF THE INVENTION

The present invention is directed to wound dressings comprising a biocompatible, hemostatic, wound contacting and/or covering substrate comprising an aldehyde-modified polysaccharide having covalently conjugated there with a hemostatic agent, for example, thrombin, fibrinogen or fibrin; and to methods of providing enhanced hemostasis to wounds.

The hemostatic wound dressings of the present invention provide and maintain effective hemostasis when applied to a wound requiring hemostasis. Effective hemostasis, as used herein, is the ability to control and/or abate capillary, venous, or arteriole bleeding within an effective time, as recognized by those skilled in the art of hemostasis.

The hemostatic dressings of the present invention are particularly useful when conventional procedures to control and/or abate bleeding, such as pressure or suturing, are either ineffective or impractical. The hemostatic wound covering substrates of the present invention comprise covalently conjugated there with hemostatic agents, or other biological or therapeutic compounds, moieties or species, particularly those "acid-sensitive" agents that may be degraded or denatured by, or otherwise detrimentally affected by acidic pH such as is provided by conventional OC hemostats.

The wound dressings may take various physical forms and may include, without limitation, fibrous or non-fibrous, knitted, woven or non-woven dressings. In preferred embodiments, the wound dressing may comprise a fiber, including microfibers, a film, a fabric, a foam, a bead, a powder, a gel, or combinations thereof. Regardless of the form of the wound dressing, it will comprise a substrate for contacting and/or covering the wound. In certain wound dressings, the dressing may consist essentially of the substrate, or may consist of the substrate. This is particularly true where the wound dressing is fabricated from a knitted, woven or non-woven hemostatic fabric that has been oxidized to provide aldehyde modification, as described herein, and which serves as the substrate for the wound dressing. In those cases, while the wound dressing may further include such components as backing layers, adhesive layers, or the like, the wound dressing can include only the hemostatic fabric.

The wound dressing substrate will comprise a wound-contacting surface. Such substrates may take various physical forms, including, but not limited to, fibrous or non-fibrous, knitted, woven or non-woven substrates. In certain embodiments, the wound dressing substrates may comprise a fiber, including microfibers, a film, a fabric, a foam, a bead, a powder, a gel, or combinations thereof. In preferred embodiments, the substrate comprises a knitted or a woven fabric. The fabric may be formed, cut or otherwise shaped to cover the wound surface, thereby providing protection of the wound from physical trauma and effective hemostasis of the wound.

Wound dressings of the present invention, and more particularly the wound-contacting substrates thereof, comprise a biocompatible, aldehyde-modified polysaccharide. In preferred wound dressings, the polysaccharide will contain an amount of aldehyde moieties effective to render the modified polysaccharide biodegradable, meaning that the polysaccharide is degradable by the body into components that either are resorbable by the body, or that can be passed readily by the body. More particularly, the biodegraded components do not elicit permanent chronic foreign body reaction because they are absorbed by the body, such that no permanent trace or residual of the component is retained at the implantation site.

Aldehyde-modified polysaccharides used in the present invention may be prepared from biocompatible polysaccharides that are useful in medical devices. Such polysaccharides include, without limitation, cellulose, alkyl cellulose, e.g. methyl cellulose, hydroxyalkyl cellulose, alkylhydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparin sulfate, dermatan sulfate, keratin sulfate, carrageenans, chitosan, starch, amylose, amylopectin, poly-N-glucosamine, polymannuronic acid, polyglucuronic acid, polyguluronic acid, and derivatives of any of the above. In preferred embodiments, the polysaccharide is oxidized as described herein to assure that the aldehyde-modified polysaccharide is biodegradable.

Such biodegrable, aldehyde-modified, regenerated polysaccharides may be represented by Structure I below. 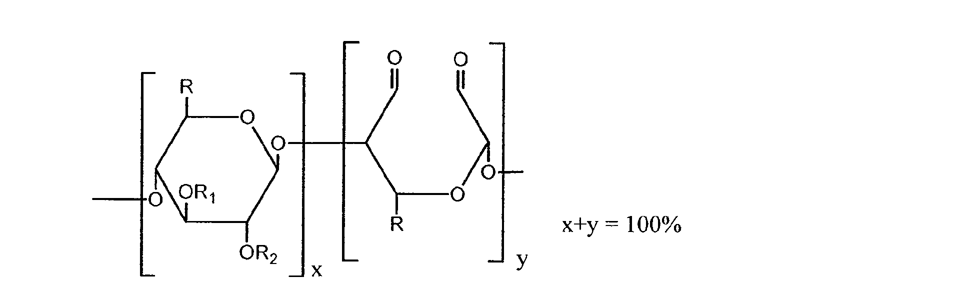
where x and y represent mole percent, x plus y equals 100 percent, x is from about 95 to about 5,
y is from about 5 to about 95; and
R may be CH₂OR₃, , COOR₄, sulphonic acid, or phosphonic acid; R₃ and R₄ may be H, alkyl, aryl, alkoxy or aryloxy, and R₁ and R₂ may be H, alkyl, aryl, alkoxy, aryloxy, sulphonyl or phosphoryl.

In preferred embodiments of the present invention, the biocompatible, biodegradable hemostatic wound dressing comprises a wound contacting/covering substrate prepared from a biocompatible, biodegradable, aldehyde-modified, regenerated polysaccharide. Regenerated cellulose is preferred due to its higher degree of uniformity versus cellulose that has not been regenerated. Regenerated cellulose is described in, for instance, United States Patent 3,364,200, the contents of which is hereby incorporated by reference as if set forth in its entirety.

In particular, preferred aldehyde-modified regenerated cellulose is one comprising repeating units of Structure II below: 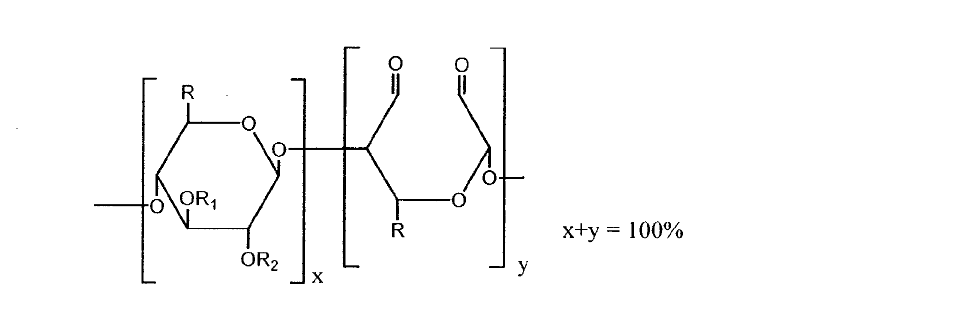
where x and y represent mole percent, x plus y equals 100 percent, x is from about 95 to about 5,
y is from about 5 to about 95; and R is CH₂OH, R₁ and R₂ are H.

In certain embodiments according to the present invention, x is from about 90 to 10 and y is about 10 to about 90. Preferably, x is from about 80 to 20 and y is from about 20 to about 80. Even more preferably, x is from about 70 to about 30. Most preferably, x is about 70 and y is about 30.

The hemostatic dressings of the present invention also provide anti-microbial activities due to the presence of effective amounts of the aldehyde moieties. It has been shown that in spite of being non-acidic, the aldehyde-modified regenerated cellulose is anti-microbial in nature. The hemostats of the present invention were found to be significantly effective against microorganisms, such as Methicillin-resistant *Staphylococcus aureus* (MRSA) *and Pseudomonas aeruginosa,* etc. The anti-microbial activities of the non-acidic aldehyde-modified regenerated cellulose are shown to be comparable to those of the acidic carboxylic oxidized regenerated cellulose conventionally used. The acidic carboxylic oxidized regenerated cellulose loses its anti-microbial activities upon neutralization reaction or over a period of time as the acid groups are neutralized in the body. However, the aldehyde-modified regenerated cellulose utilized in the present invention is expected to retain its anti-microbial activity over a longer period of time.

In preferred embodiments of the invention, the aldehyde-modified regenerated polysaccharide is essentially free of functional or reactive moieties other than aldehyde moieties. By essentially free, it is meant that the polysaccharide does not contain such functional or reactive moieties in amounts effective to alter the properties of the aldehyde-modified polysaccharide or to provide the substrate comprising the polysaccharide with a pH of less than about 4.5, more preferably less than about 5, or greater than about 9, preferably about 9.5. Such moieties include, without limitation, carboxylic acid moieties typically present on wound dressings made from OC. Excess levels of carboxylic acid moieties will lower the pH of the substrates and dressings so that they are not compatible for use with those acid sensitive species that may be degraded or denatured by such a low pH, e.g. thrombin. Other moieties include, without limitation, sulfonyl or phosphonyl moieties.

The hemostat of the present invention exhibits increased thermal stability compared to that of the carboxylic oxidized regenerated cellulose fabric (ORC). The increased thermal stability may be indicative of improved physical shelf-life, compared to ORC or neutralized ORC.

In certain embodiments of the invention, the fabrics utilized in the present invention may be knitted, woven or non-woven, provided that the fabric possesses the physical properties adequate for wound dressings, in general, and hemostatic wound dressings, specifically. Fabrics oxidized by periodic acid or its salts described in the present invention are expected to retain physical properties and mechanical integrity required for use in wound dressings. Hemostatic fabrics useful for use in hemostatic wound dressings according to the present invention include fabrics comprising the aldehyde-modified polysaccharides of the present invention and being of the structure described in United States Patent Numbers 2,773,000, 3,364,200, 4,626,253, and 5,002,551, the contents each of which is hereby incorporated by reference herein as if set forth in its entirety.

In certain embodiments of the invention, the hemostatic wound dressing of the present invention comprises as the wound contacting/covering hemostatic substrate a warp knitted tricot fabric constructed of bright rayon yarn that has been oxidized by periodic acid or its salts such that the substrate comprises aldehyde moieties. Both Scanning Electron Microscopic (SEM) images and fabric mechanical properties indicate that the physical characteristics (density, thickness) and physical performance, e.g. fabric tensile strength and Mullen burst strength, of the aldehyde-modified regenerated cellulose in the present invention are comparable to those of the fabric disclosed in United States Patent 4,626,253.

The hemostat of the present invention remains very flexible, conforms to a bleeding site, and retains good tensile and compressive strength to withstand handling during application. The aldehyde-modified regenerated cellulose hemostat can be cut into different sizes and shapes to fit the surgical needs. It can be rolled up or packed into irregular anatomic areas.

Other warp knit tricot fabric constructions which produce equivalent physical properties may, of course, be utilized in the manufacture of the aldehyde-modified regenerated cellulose hemostatic wound dressings of the present invention, and such constructions will be apparent to those skilled in the art once having the benefit of this disclosure.

In other embodiments, the hemostat of the present invention comprises of powdered or pulverized aldehyde-modified regenerated cellulose fabric conjugated with the hemostatic agents.

In certain embodiments of the invention, a biologics, a drug or a combination of pharmaceutical agents that otherwise may be sensitive to the low pH of OC-containing wound dressings, such agents may be incorporated into certain wound dressings of the present invention without having to adjust pH prior to incorporation into the dressing. To fabricate such a hemostatic wound dressing, a drug or agent is first dissolved in an appropriate solvent. The fabric is then coated with the drug solution, and the solvent is removed. Preferred biologics, drugs and agent include analgesics, anti-infective agents, antibiotics, adhesion preventive agents, pro-coagulants, and wound healing growth factors.

The aldehyde groups formed on the polysaccharide matrix during the periodate oxidation reaction can be used to covalently bond amine containing biologics and therapeutic agents. The combination of such biologics, drugs and agents with wound dressings of the present invention using the aldehyde-modified regenerated cellulose substrates can provide improved hemostatic wound dressings, wound healing dressings, drug delivery devices, and tissue engineering matrices.

Substrates used in wound dressings of the present invention comprise an aldehyde-modified polysaccharide comprising covalently conjugated there with a hemostatic agent bearing an aldehyde reactive moiety. The hemostatic agent, including procoagulant enzymes, proteins and peptides, can be naturally occurring, recombinant, or synthetic, and may be selected from the group consisting of prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin and vasopressin analogs, epinephrine, selectin, procoagulant venom, plasminogen activator inhibitor, platelet activating agents, synthetic peptides having hemostatic activity, and any combination thereof. Preferred hemostatic agents in the present invention are thrombin, fibrinogen and fibrin.

The aldehyde moiety of aldehyde-modified regenerated polysaccharide can readily react with the amine groups present on the amino acid side chains or N-terminal residues of thrombin, fibrinogen or fibrin, resulting in forming a conjugate of the hemostatic agent with the aldehyde-modified regenerated polysaccharide covalently linked by a reversible imine bond. The imine bonded aldehyde-modified regenerated polysaccharide/hemostatic agent conjugate may then be further reacted with a reducing agent such as sodium borohydride or sodium cyanoborohydride to form an irreversible secondary amine linkage. In preferred embodiments of the invention, the hemostatic agent is dispersed at least on the wound-contacting surface of the substrate, and preferably at least partially through the wound contacting substrate, bonded covalently to the aldehyde-modified polysaccharide by reversible or irreversible bonds.

Oxidation of 2, 3- vicinal hydroxyl groups in a carbohydrate with periodic acid (or any alkali metal salt thereof) forms a di-aldehyde or di-aldehyde derivatives. These aldehyde moieties(-RCH(O)) can then readily react with a primary amine moiety (-NH₂), such as are present on the amino acid side chains or N-terminal residues of proteins, resulting in an equilibrium with the reaction product, a protein and carbohydrate conjugate, covalently linked by a relatively unstable and reversible imine moiety (-N=CHR). To stabilize the linkage between the biomolecule and the substrate surface, subsequent reductive alkylation of the imine moiety is carried out using reducing agents (i.e., stabilizing agents) such as, for example, sodium borohydride, sodium cyanoborohydride, and amine boranes, to form a secondary amine (-NH-CH₂-R) .

As noted above, wound dressings of the present invention provide rapid hemostasis and maintain effective hemostasis in cases of severe bleeding. Examples of severe bleeding include, without limitation, arterial puncture, liver resection, blunt liver trauma, blunt spleen trauma, aortic aneurysm, bleeding from patients with over-anticoagulation, or bleeding from patients with coagulopathies, such as hemophilia. Protein based hemostatic agents, such as thrombin, fibrin or fibrinogen, if covalently conjugated to the aldehyde groups of the aldehyde-modified polysaccharide to form a secondary amine linkage by converting the imine bond with reducing agents such as sodium borohydride or sodium cyanoborohydride bond, can enhance the hemostatic property of aldehyde-modified regenerated cellulose wound dressings and reduce the risk of thrombosis caused by free hemostatic agents migrating into the blood stream.

The hemostatic wound dressing of the present invention comprises hemostatic agents, including but not limited to thrombin, fibrinogen or fibrin, in an amount effective to provide rapid hemostasis and maintain effective hemostasis in cases of severe bleeding. If the concentration of the hemostatic agent on the aldehyde-modified regenerated cellulose substrate is too low, the hemostatic agents do not provide an effective proagulant activity to promote rapid clot formation upon contact with blood or blood plasma. A preferred concentration range of thrombin on aldehyde-modified regenerated cellulose substrate is from about 0.001 to about 1 percent by weight. A more preferred concentration of thrombin on aldehyde-modified regenerated cellulose substrate is from about 0.01 to about 0.1 percent by weight. A preferred concentration range of fibrinogen on the aldehyde-modified regenerated cellulose substrate is from about 0.1 to about 50 percent by weight. A more preferred concentration of fibrinogen on the aldehyde-modified regenerated cellulose substrate is from about 2.5 to about 10 by weight. A preferred concentration range of fibrin on the aldehyde-modified regenerated cellulose substrate is from about 0.1 to about 50 percent by weight. A more preferred concentration of fibrin on the aldehyde-modified regenerated cellulose substrate is from about 2.5 to about 10 by weight.

The features of such covalently bonded hemostatic agents conjugated with the aldehyde-modified regenerated cellulose wound dressing can be controlled to suit a desired application by choosing the conditions to form the composite hemostat during conjugation.

In certain embodiments of the present invention, the hemostatic agent, such as thrombin, fibrinogen or fibrin, is dispersed substantially homogeneously through the wound dressing substrate. In such cases, aldehyde-modified regenerated cellulose substrate may be immersed in the solution of thrombin, fibrinogen or fibrin to provide homogeneous distribution throughout the wound dressing.

In other embodiments of the present invention, a faster hemostat can be created by the following procedure. The aldehyde-modified regenerated cellulose wound dressing can be soaked with the desired amount of aqueous solution of thrombin and rapidly lyophilized using known methods that retain therapeutic activity. The dry hemostatic biologic conjugate can be used as a fast hemostat with excellent bactericidal activity, biodegradability, bioabsorbability and long-lasting stability.

In other embodiments, it is preferred that aldehyde-modified regenerated cellulose substrate is soaked with a solution of fibrinogen and subsequently exposed to thrombin prior to lyophilization.

In certain embodiments of the invention, the thrombin conjugate of aldehyde-modified regenerated cellulose substrate is further reacted with reducing agents such as sodium borohydride or sodium cyanoborohydride to form a secondary amine linkage. The aldehyde-modified regenerated cellulose substrate can be soaked with the desired amount of aqueous solution of thrombin, then reacted with aqueous solution of sodium borohydride or sodium cyanoborohydride reconstituted in phosphate buffer (PH=8) prior to lyophilization.

The reduced form of the aldehyde-modified regenerated cellulose-thrombin conjugate is more stable due to the nature of the secondary amine linkage. Hemostatic wound dressings of this embodiment have enhanced hemostatic properties, as well as increased stability, and can provide rapid hemostasis without causing thrombin to migrate into the blood stream and cause severe thrombosis.

In other embodiments of the present invention, it is preferred that thrombin is constituted in an aqueous solution of a non-acidic water-soluble polymer, including but not limited to alkyl cellulose, e.g. methyl cellulose, hydroxyalkyl cellulose, alkyl hydroxyalkyl cellulose, salts of carboxymethyl or carboxyethyl cellulose, chitin, salts of hyaluronic acid, alginate, propylene glycol alginate, glycogen, dextran, carrageenans, chitosan, starch, amylose, and poly-N-glucosamine. The aldehyde-modified regenerated cellulose wound dressing can be soaked with the desired amount of aqueous solution of thrombin and the water-soluble polymer and rapidly lyophilized using known methods that retain therapeutic activity. The dry hemostatic biologic conjugate patch can be used as a fast hemostat.

In certain embodiments of the invention, a biologic, a drug or a combination of pharmaceutical agents can be incorporated into the hemostat without adjusting it pH value. Preferred agents include but not limited to analgesics, anti-infective agents, antibiotics, adhesion preventive agents, procoagulants, and wound healing growth factors. To construct such a hemostat, a pharmaceutical agent is first dissolved in an appropriate solvent. The wound dressing is then coated with such solution, and the solvent is removed. The combination of such biologics, drugs and agents with the aldehyde-modified oxidized regenerated cellulose hemostat of the present invention can construct faster hemostat, better wound healing device, drug delivery device, and tissue engineering matrix.

While the following examples demonstrate certain embodiments of the invention, they are not to be interpreted as limiting the scope of the invention, but rather as contributing to a complete description of the invention. Treatment times and temperatures for reactions in the examples below tend to be inversely related. Higher temperatures require relatively shorter treatment times. The limitations of the time and temperature are governed by the effect on the biological stability of the hemostatic agents. Conditions outside what is described below are still within the scope of this invention.

### Example 1:

### Preparation of knitted aldehyde-modified regenerated cellulose fabric:

A 15.75 g piece of Nu-Knit® rayon fabric was cut in the form of a strip 1.5 inches wide. The strip was wound on a mandrel and suspended in 600 ml of aqueous isopropyl alcohol (IPA) (200 ml IPA/400 ml de-ionized (DI) water). 20.8 g of sodium periodate (Aldrich, Milwaukee, 53201) was dissolved in the solution (1:1 molar ratio) and the mandrel was rotated at moderate rpm in the solution for 21 hours at ambient temperature. It is essential that the oxidation of the fabric be conducted in the dark. The solution pH was 3.8. The solution was discarded after the reaction. The mandrel with the oxidized fabric was washed for 30 minutes in 1 liter of cold DI water containing 50 ml of ethylene glycol. It was then washed with aqueous IPA (50/50) for 15 minutes, followed by a pure IPA wash for 15 minutes. The fabric was dried in ambient air for several hours. [Aldehyde content: Ave. 22.83%]

The oxidized fabric then was evaluated for hemostasis as set forth below. Results are provided in Table 1.

### Example 2:

### Preparation of non-woven aldehyde-modified cellulose fabric:

A 10 g piece of cellulose rayon non-woven fabric was cut in the form of a rectangle and placed in an aqueous solution of sodium periodate (Aldrich, Milwaukee, 53201) (1:0.7 molar ratio). The fabric was placed in a container modified to exclude light and soaked in the dark for 24 hours at 37°C. The solution was discarded after the reaction. The fabric was repeatedly washed with DI water until the pH was 6-7. It was then washed with aqueous IPA (50/50) for 15 minutes. The fabric then was washed in pure IPA for 15 minutes. The fabric was dried in ambient air for several hours. [aldehyde content: 51.04%]

The oxidized fabric then was evaluated for hemostasis as set forth below. Results are provided in Table 1.

### Example 3:

### Preparation of aldehyde-modified regenerated cellulose powders:

10.6 g of powdered cellulose rayon was suspended in an aqueous solution of sodium periodate (Aldrich, Milwaukee, 53201)(13.9 g in 250 ml DI water] and stirred for 7 hours at ambient temperature in the dark. The solution was filtered after the reaction. The filtrate was repeatedly washed with DI water until the pH was in the range of from 6 to 7. It was then washed with aqueous IPA (50/50) and pure IPA for 15 min each. The powder was dried in air for several hours. [aldehyde content: 32.8 %]

The oxidized powder then was evaluated for hemostasis as set forth below. Results are provided in Table 1.

### Example 4:

### Preparation of aldehyde-modified cellulose beads:

13.67 g of porous cellulose beads are floated in an aqueous solution of sodium periodate (Aldrich, Milwaukee, 53201) (18g in 250 ml DI water/125ml IPA) and stirred for 24 hours at ambient temperature. The material was filtered and the filtrate (beads and crushed beads) was repeatedly washed with DI water until the pH was in the range of from 6 to 7. It was then washed with aqueous IPA (50/50) and pure IPA for 15 min each. The material was dried in air for several hours. [aldehyde content: intact beads-29.86 %; crushed beads-35%]

Thrombin conjugates with the oxidized beads were prepared similar to methods disclosed herein. The oxidized beads and thrombin conjugates then were evaluated for hemostasis as set forth below. Results are provided in Table 1.

### Example 5:

### Thrombin conjugated with aldehyde-modified regenerated cellulose

An 8 g piece of fabric prepared in Example 1 was soaked in 20 ml of freshly reconstituted thrombin solution (1000 units/ml) in a flat metal pan. The thrombin solution accordingly was distributed throughout the fabric substrate. The pan was quickly introduced into a pre-cooled freezer maintained at -20°C. The material was stored frozen. The pan was transferred into a " Virtis Advantage" lyophilizer with a shelf-temperature of -50°C. The pan was maintained at that temperature under vacuum for 6 hours. The temperature was raised and maintained at -15°C for another 2 hours. It was then subsequently raised to 0°C and 15°C for 16 hours at each temperature. At this time the water had completely sublimed. The vacuum was released and the fabric was removed from the pan. The thrombin, covalently conjugated with the aldehyde-modified regenerated cellulose, was distributed throughout the substrate via the lyophilization of the fabric in solution. The flexible material was stored in the refrigerator in an airtight container until further use. A portion of the lyophilized fabric conjugate was pulverized into a powder.

The thrombin-conjugated aldehyde-modified regenerated cellulose fabric then were evaluated for hemostasis as set forth below. Results are provided in Table 1.

### Example 6:

### Thrombin conjugated with aldehyde-modified regenerated cellulose and immobilized by reduction.

A 3.2 g piece of fabric prepared according to Example 1 was soaked in 8 ml of thrombin solution in phosphate buffer (pH = 8) at 800 units/ml in a flat metal pan ('A'). In another pan ('B'), 2.9 g of the same fabric was similarly soaked with 8 ml of the thrombin solution. Both pans were quickly introduced into a pre-cooled freezer maintained at -20°C. After 13 hours, pan 'A' was thawed and the wet fabric was quickly transferred into a large centrifuge tube containing 45 ml of (50 mM) NaCNBH₄ reconstituted in phosphate buffer (pH 8). The fabric was completely submerged in the solution for 15 min. The fabric was isolated and repeatedly washed with DI water. The final wet fabric was placed on the pan and frozen at -20°C. Both pans were quickly transferred into a 'Virtis Advantage' lyophilzer with a shelf-temperature of -50°C. They were maintained at that temperature under vacuum for 2 hours. The temperature was raised and maintained at -15°C for another 12 hours. It was then subsequently raised to 0°C and 15°C for 2 hours at each temperature. At this time the water had completely sublimed. The vacuum was released and the fabrics were removed from the pan. The flexible materials were stored in the refrigerator in an airtight container until further use.

### Example 7:

### Fibrinogen conjugated with aldehyde-modified regenerated Cellulose

An 8 g piece of fabric as produced according to Example 1 was soaked in 20 ml of freshly reconstituted fibrinogen solution (40 mg/ml) in a flat metal pan. The fabric was lyophilized and, as before, and a portion pulverized as in Example 5. The fabric was evaluated and was evaluated for hemostasis as set forth below. Results are provided in Table 1.

### Example 8:

### Fibrin conjugated with aldehyde-modified regenerated Cellulose

An 8 g piece of fabric according to Example 1 was soaked in 20 ml of freshly reconstituted fibrinogen solution (40 mg/ml) in a flat metal pan. This was sprayed with an equal amount of thrombin solution (1000 unit/ml). A gel was rapidly formed. The pan was quickly introduced and stored in a pre-cooled freezer maintained at -20°C. The pan was subsequently transferred into a 'Virtis Advantage' lyophilzer with a shelf-temperature of -50°C. The pan was maintained at that temperature under vacuum for 2 hours. The temperature was raised and maintained at -15°C for another 12 hours. It was then subsequently raised to 0°C and 15°C for 2 hours at each temperature. At this time the water had completely sublimed. The vacuum was released and the fabric was removed from the pan. The flexible material was stored in the refrigerator in an airtight container under further use.

The fibrin conjugated aldehyde-modified regenerated cellulose fabric then was evaluated for hemostasis as set forth below. Results are provided in Table 1.

### Example 9:

### Blends of powder conjugates.

Pulverized conjugates as prepared in Examples 5 and 7 were blended and evaluated for hemostatis as set forth below. Results are presented in Table 1.

### Example 10:

### Hemostatic performance of different materials in porcine splenic incision model

A porcine spleen incision model was used for hemostasis evaluation of different materials. The materials were cut into 2.5 cm X 2.0 cm rectangles. A linear incision of 1.5 cm with a depth of 1.0 cm was made with a surgical blade on a porcine spleen. After application of the test article, digital tamponade was applied to the incision for 2 minutes. The hemostasis was then evaluated. Additional applications of digital tamponade for 30 seconds each time were used until complete hemostasis was achieved. Fabrics failing to provide hemostasis within 12 minutes were considered to be failures. Wound dressings comprising aldehyde-modified regenerated cellulose achieve rapid hemostasis compared to the negative control of surgical gauze, as shown in table 1. Observations on effectiveness of thrombin, fibrinogen and fibrin as hemostatic agents in reducing time to hemostasis are also shown in table 1.

**Table 1**

| Hemostatic performance of Aldehyde-Modified Regenerated Cellulose (AMRC) Based-Materials | | |
|---|---|---|
| Example No. | Sample | Time to Hemostasis (seconds) |
| 1 | AMRC knitted fabric | 187 (n=11) |
| 5 | AMRC/Thrombin (fabric) | 30 (n=3) |
| 8 | AMRC /fibrin (fabric) | 30 (n=4) |
| 7 | AMRC/fibrinogen (fabric) | 65 (n=2) |
| 2 | AMRC Non-woven fabric | 96 (n=5) |
| 3 | AMRC powder | 120 (n=3) |
| 5 | AMRC/Thrombin (powder) | 30 (n=3) |
| 8 | AMRC/fibrin (powder) | 30 (n=1) |
| 9 | AMRC/thrombin powder plus AMRC/fibrinogen powder | 250 (n=1) |
| 4 | AMRC Beads | 238 (n=1) |
| 4 | AMRC Beads/Thrombin | 30 (n=3) |
| | Surgical gauze Control | >720 (n=6) |

## Claims

1. A hemostatic wound dressing, comprising:
a substrate for contacting a wound, said substrate comprising,
a wound-contacting surface,
a biocompatible aldehyde-modified polysaccharide; and
a hemostatic agent covalently conjugated with said aldehyde-modified polysaccharide, said agent comprising an aldehyde-reactive moiety,
wherein said wound dressing is hemostatic.

2. The wound dressing of claim 1 wherein said substrate comprises a fiber, a fabric, a sponge, a foam, a film, a bead, a gel, a powder, or combinations thereof

3. The wound dressing of claim 1 wherein said aldehyde-modified polysaccharide is selected from the group consisting of aldehyde-modified cellulose, alkyl cellulose, hydroxyalkyl cellulose, alkylhydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparin sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

4. The wound dressing of claim 3 wherein said aldehyde-modified polysaccharide comprises an amount of aldehyde effective to render the polysaccharide biodegradable.

5. The wound dressing of claim 4 wherein said aldehyde-modified polysaccharide is selected from the group consisting of aldehyde-modified starch, dextran, pectin, alginate, chitin, chitosan, glycogen, amylose, amylopectin, cellulose and cellulose derivatives.

6. The wound dressing of claim 5 wherein said aldehyde-modified polysaccaride comprises aldehyde-modified regenerated polysaccharide.

7. The wound dressing of claim 6 wherein said aldehyde-modified polysaccharide comprises aldehyde-modified regenerated cellulose comprising repeating units of structure II, 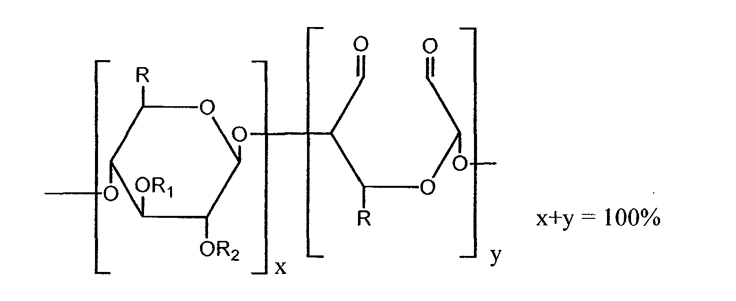
wherein x plus y equals 100 percent, x ranges from about 95 to about 5 percent, and
y ranges from about 5 to about 95 percent and R is CH₂OH, and R₁ and R₂ are H.

8. The wound dressing of claim 7 wherein x ranges from about 80 to about 20 percent and y ranges from about 20 to about 80 percent.

9. The wound dressing of claim 8 wherein x is about 70 percent and y is about 30 percent.

10. The wound dressing of claim 1 wherein said aldehyde-modified polysaccharide is essentially free of carboxylic acid.

11. The wound dressing of claim 7 wherein said aldehyde-modified cellulose is essentially free of carboxylic acid.

12. The wound dressing of claim 1 wherein said hemostatic agent is synthetic, recombinant or naturally occurring.

13. The wound dressing of claim 1 wherein said hemostatic agent is selected from the group consisting prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin, vasopressin analogs, epinephrine, selectin, procoagulant venom, plasminogen activator inhibitor, platelet activating agents and synthetic peptides having hemostatic activity.

14. The wound dressing of claim 1 wherein said substrate comprises from about 0.001 to about 50 percent by weight of said hemostatic agent.

15. The wound dressing of claim 11 wherein said substrate comprises from about 0.001 to about 1 percent by weight of thrombin as the hemostatic agent.

16. The wound dressing of claim 15 wherein said substrate comprises from about 0.01 to about 0.1 percent by weight of thrombin as the hemostatic agent.

17. The wound dressing of claim 11 wherein said substrate comprises from about 0.1 to about 50 percent by weight of fibrinogen as the hemostatic agent.

18. The wound dressing of claim 17 wherein said substrate comprises from about 2.5 to about 10 percent by weight of fibrinogen as the hemostatic agent.

19. The wound dressing of claim 11 wherein the substrate comprises from about 0.1 to about 50 percent by weight of fibrin as the hemostatic agent.

20. The wound dressing of claim 19 wherein the substrate comprises from about 2.5 to about 10 percent by weight of fibrin as the hemostatic agent.

21. The wound dressing of claim 1 wherein said hemostatic agent is dispersed at least partially through said substrate.

22. The wound dressing of claim 1 wherein said hemostatic agent is conjugated with said aldehyde-modified polysaccharide by covalent imine bonding.

23. The wound dressing of claim 1 wherein said hemostatic agent is conjugated with said aldehyde-modified polysaccharide by covalent secondary amine linkage.

24. A method of providing hemostasis to a wound, comprising:
applying to a wound a hemostatic wound dressing, comprising:
a substrate for contacting a wound, said substrate comprising,
a wound-contacting surface,
a biocompatible aldehyde-modified polysaccharide; and
a hemostatic agent covalently conjugated with said aldehyde-modified polysaccharide, said agent comprising an aldehyde-reactive moiety,
wherein said wound dressing is hemostatic.

25. The method of claim 24 wherein said substrate comprises a fiber, a fabric, a sponge, a foam, a film, a bead, a gel, a powder, or combinations thereof

26. The method of claim 24 wherein said aldehyde-modified polysaccharide is selected from the group consisting of aldehyde-modified cellulose, alkyl cellulose, hydroxyalkyl cellulose, alkylhydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparin sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

27. The method of claim 26 wherein said aldehyde-modified polysaccharide comprises an amount of aldehyde effective to render the polysaccharide biodegradable.

28. The method of claim 27 wherein said aldehyde-modified polysaccaride comprises aldehyde-modified regenerated polysaccharide.

29. The wound dressing of claim 27 wherein said aldehyde-modified polysaccharide comprises aldehyde-modified regenerated cellulose comprising repeating units of structure II, 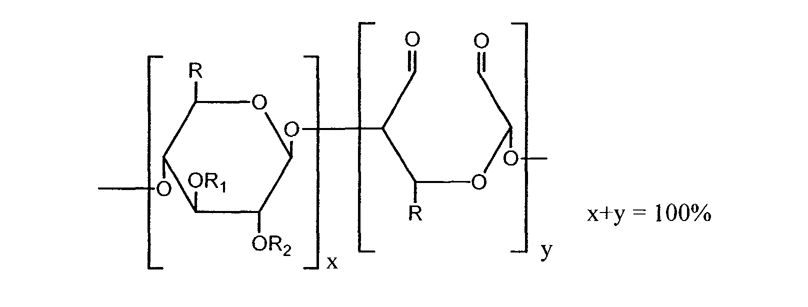
wherein x plus y equals 100 percent, x ranges from about 95 to about 5 percent, and
y ranges from about 5 to about 95 percent and R is CH₂OH, and R₁ and R₂ are H.

30. The method of claim 29 wherein x ranges from about 80 to about 20 percent and y ranges from about 20 to about 80 percent.

31. The method of claim 24 wherein said aldehyde-modified polysaccharide is essentially free of carboxylic acid.

32. The method of claim 29 wherein said aldehyde-modified cellulose is essentially free of carboxylic acid.

33. The wound of claim 24 wherein said hemostatic agent is synthetic, recombinant or naturally occurring.

34. The method of claim 33 wherein said hemostatic agent is selected from the group consisting prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin, vasopressin analogs, epinephrine, selectin, procoagulant venom, plasminogen activator inhibitor, platelet activating agents and synthetic peptides having hemostatic activity.

35. The method of claim 24 wherein said substrate comprises from about 0.001 to about 50 percent by weight of said hemostatic agent.

36. The method of claim 29 wherein said substrate comprises from about 0.001 to about 1 percent by weight of thrombin as the hemostatic agent.

37. The method of claim 29 wherein said substrate comprises from about 0.1 to about 50 percent by weight of fibrinogen as the hemostatic agent.
